# EUROPEAN PATENT SPECIFICATION

(11) **EP 4 178 668 B1**
(45) Date of publication and mention of the grant of the patent: **22.10.2025**
(21) Application number: 21748736.2
(22) Date of filing: 06.07.2021
(51) Int. Cl.: A61N 1/375, H01R 24/00

(54) **COMPOSITE HEADER SEALS**
VERBUND-HEADERDICHTUNGEN
JOINTS D'EMBASE COMPOSITES

(30) Priority: 07.07.2020 US 202063048857 P
(43) Date of publication of application: 17.05.2023
(73) Proprietor: Cardiac Pacemakers, Inc., St. Paul, Minnesota 55112 (US)
(72) Inventor: ENGLISH, James M., Tipperary Cahir (IE); SWEENEY, Moira B., St. Paul, Minnesota 55104 (US); JONES, Robert A., Lake Elmo, Minnesota 55042 (US); HAASL, Benjamin J., Forest Lake, Minnesota 55025 (US); O'ROURKE, John, Clonmel, (IE); ACHTERHOFF, Trey H., St Paul, Minnesota 55104 (US); FOSTER, Arthur J., Blaine, Minnesota 55449 (US)
(74) Representative: Vossius & Partner Patentanwälte Rechtsanwälte mbB
(86) International application number: PCT/US2021/040464
(87) International publication number: WO 2022/010861

(56) References cited:
- US-A1- 2003 163 171
- US-A1- 2007 225 772
- US-A1- 2008 246 231
- US-A1- 2010 204 740
- US-B2- 6 755 694

## Description

### TECHNICAL FIELD

The present description relates to an implantable system having an implantable lead having a connector port. More specifically, the description relates to a connector port and components arranged within the connector port that provide a simplified core assembly. The present invention is set out in the appended claims.

### BACKGROUND

implantable medical systems for stimulating a target area or for diagnostic purposes may include different lead configurations that require different industry standards. The systems may include an implantable lead assembly and an implantable pulse generator connected with the implantable lead assembly. The implantable lead assembly may comply with one or more of industry standards (e.g., IS-1, IS4, DF4). Further, a header of the implantable pulse generator generally includes corresponding connector ports that are configured to comply with one or more of the standards so that the implantable lead assembly may be effectively coupled with the implantable pulse generator. A proper connection between the implantable leads and the corresponding connector ports is required to allow proper functioning of the implantable system. Documents US 2007/225772 and US 2008/256231 disclose known connector port subassemblies.

### SUMMARY

The present invention pertains to a connector port subassembly as defined in claim 1 and corresponding manufacturing method according to claim 10.

Embodiments according to the present teaching will become apparent to those skilled in the art from the following detailed description, which shows and describes illustrative embodiments Accordingly, the drawings and detailed description are to be regarded as illustrative in nature and not restrictive.

### BRIEF DESCRIPTION OF THE DRAWINGS

FIG. 1 is an illustration of an example implantable system for stimulating a target location on or within the heart, in accordance with various aspects of the present disclosure.
FIG. 2A is a perspective illustration of a seal ring arranged within a core section of a connector port subassembly medical device, in accordance with various aspects of the present disclosure.
FIG. 2B is a cross-section illustration of the seal ring and core section, shown in FIG. 2A, in accordance with various aspects of the present disclosure.
FIG. 3 is a cross-sectional illustration of an example connector port subassembly medical device, in accordance with various aspects of the present disclosure.
FIG. 4 is a cross-sectional illustration of an illustration of an example seal ring and core section, in accordance with various aspects of the present disclosure.
FIG. 5 is a cross-sectional perspective illustration of illustration of another example seal ring, in accordance with various aspects of the present disclosure.

While the present teaching is amenable to various modifications and alternative forms, specific embodiments have been shown by way of example in the drawings and are described in detail below. The intention, however, is not to limit the present teaching to the particular embodiments described. On the contrary, the present teaching is intended to cover all modifications, equivalents, and alternatives falling within the scope of the present disclosure.

### DETAILED DESCRIPTION

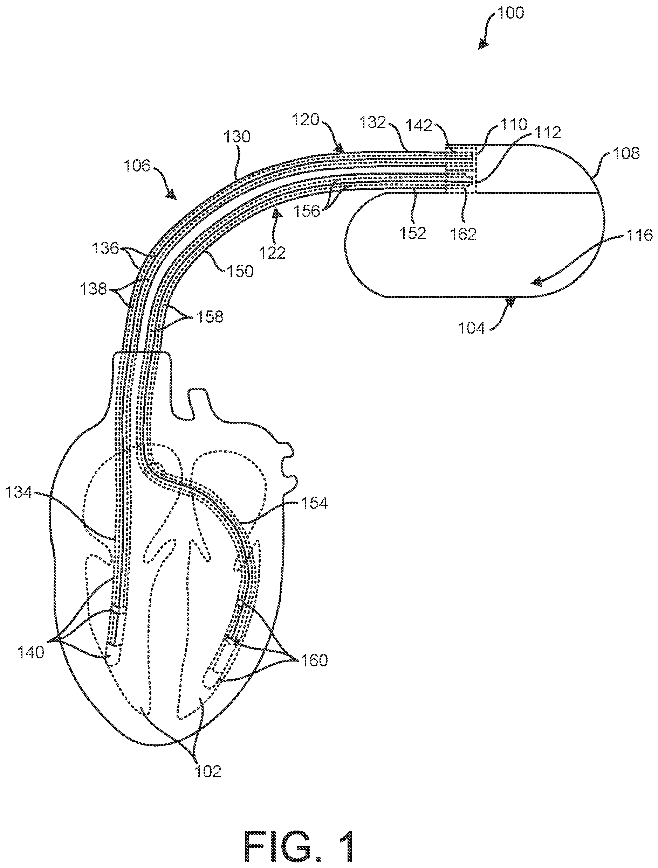

FIG. 1 is a schematic illustration of an implantable system 100 for stimulating a target location 102 on or within the heart. As shown, the implantable system 100 includes an implantable medical device (IMD) 104 and an implantable lead assembly 106 connected to the IMD 104. In various embodiments, the IMD 104 is an implantable pulse generator adapted to generate electrical signals to be delivered to the target location 102 for pacing and/or for sensing electrical activity at a location on or within the heart. The IMD 104 can include microprocessors to provide processing, evaluation, and to deliver electrical shocks and pulses of different energy levels and timing for defibrillation, cardioversion, and pacing to a heart in response to cardiac arrhythmia including fibrillation, tachycardia, heart failure, and bradycardia. In other instances, the implantable system 100 can also be suitable for use with implantable electrical stimulators, such as, but not limited to, neuro-stimulators, skeletal stimulators, central nervous system stimulators, or stimulators for the treatment of pain.

The IMD 104 may include one or more connector ports 110, 112. In certain instances, the IMD (*e*.*g*., pulse generator 104) includes a header 108 with the connector port(s) 110, 112. As shown, for example, the header 108 includes a first connector port 110 and a second connector port 112. In addition, the implantable lead assembly 106 includes a first implantable lead 120 connected to the first connector port 110 and a second implantable lead 122 connected to the second connector port 112. In some instances, the implantable lead assembly 106 may also include a third implantable lead (not shown) and the header 108 may include a corresponding third connector port (not shown and up to five connector ports may be used). The IMD 104 also includes a housing 116 that is connected to the header 108. The housing 116 can include a source of power as well as electronic circuitry. The header 108 may be overmoulded to the housing 116 and may be formed of a rigid polymer that is a non-conductive polymer such as, for example, an aromatic polyether-based thermoplastic polyurethane, polyether ether ketone, epoxy, or a polyethersulfone.

Each of the first and second implantable leads 120, 122 includes a flexible lead body, a plurality of conductor wires, a plurality of electrodes, and a terminal connector assembly. For example, as shown, the first implantable lead 120 includes a flexible lead body 130 having a proximal end 132, a distal end portion 134, and a plurality of conductor lumens 136 extending axially within the flexible lead body 130 from the proximal end 132 to the distal end portion 134. The first implantable lead 120 also includes a plurality of conductor wires 138, each conductor wire extending within one of the conductor lumens 136 in the flexible lead body 130. The first implantable lead 120 further includes a plurality of electrodes 140 coupled to the distal end portion 134 of the flexible lead body 130. Each of the electrodes 140 is electrically coupled to at least one of the plurality of conductor wires 138. The first implantable lead 120 also includes a terminal connector assembly 142 (or terminal pin) coupled to the proximal end 132 of the flexible lead body 130. The terminal connector assembly 142 is sized to be inserted into and received by the first connector port 110 of the header 108.

Similarly, the second implantable lead 122 includes a flexible lead body 150 having a proximal end 152, a distal end portion 154, and a plurality of conductor lumens 156 extending axially within the flexible lead body 150 from the proximal end 152 to the distal end portion 154. The second implantable lead 122 also includes a plurality of conductor wires 158, each conductor wire extending within one of the conductor lumens 156 in the flexible lead body 150. Further, the second implantable lead 122 includes a plurality of electrodes 160 coupled to the distal end portion 154 of the flexible lead body 150. Each of the electrodes 160 is electrically coupled to at least one of the plurality of conductor wires 158. The second implantable lead 122 also includes a terminal connector assembly 162 coupled to the proximal end 152 of the flexible lead body 150. The terminal connector assembly 162 is sized to be inserted into and received by the second connector port 112 of the header 108.

As an example of implant locations for one or more leads, the first implantable lead 120 is shown extending into a right ventricle of the heart, and the second implantable lead 122 extending through the coronary sinus and into a coronary vein disposed outside the left ventricle of the heart. The electrical signals and stimuli conveyed by the IMD 104 are carried to the electrode at the distal end of the lead by the conductors. The IMD 104 is typically implanted subcutaneously within an implantation location or pocket in the patient's chest or abdomen. As shown in FIG. 1, the header 108 may include one or more connector port(s) 110, 112. Components arranged within the connector port(s) 110, 112 form a subassembly that differs based on the industry standard of the lead(s) 120, 122 that is to be inserted into the connector port(s) 110, 112.

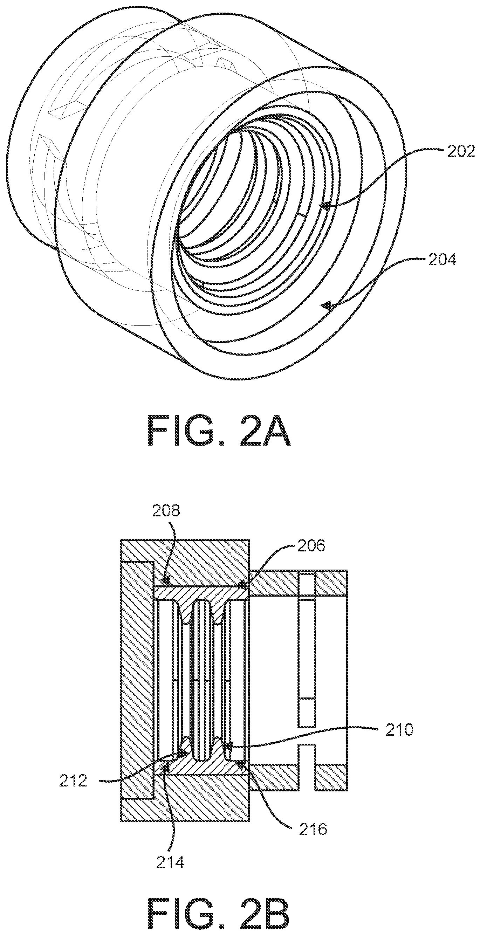

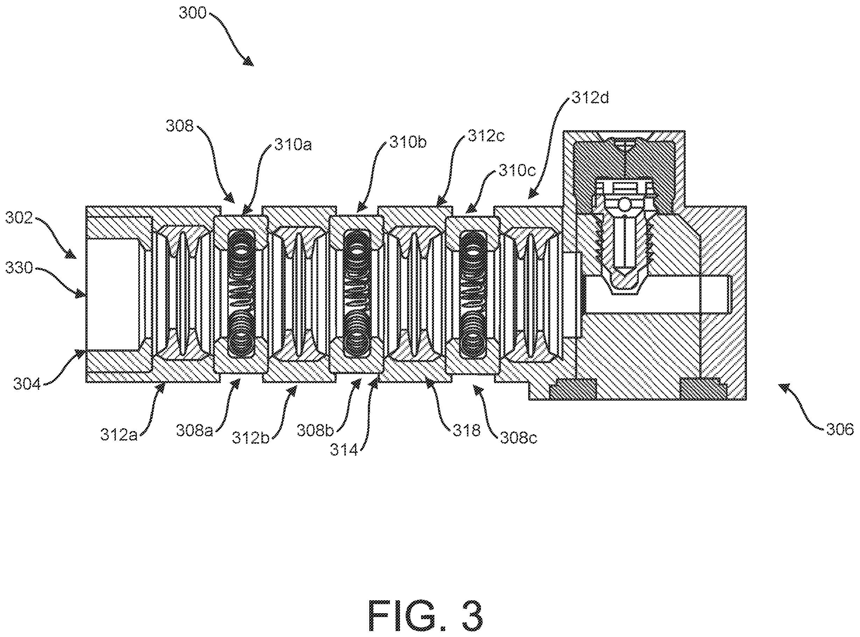

FIG. 2A is a perspective illustration of a seal ring 202 arranged within a core section 204 of a connector port subassembly medical device, in accordance with various aspects of the present disclosure. The core section 204 may form a portion of a connector port subassembly as shown in further detail in FIG. 3. The core section 204 may be one of a plurality of core sections 204 that form a connector port subassembly. Similarly, the seal ring 202 may be one of a plurality of seal rings 202 arranged within a connector port subassembly.

In certain instances, the seal ring 202 includes two or more materials. The seal ring 202 may be formed of a moulded composite of the two or more materials. The two or more materials may include a first material and a second material with the first material and the second material being a common material (e.g., silicone, a polymer material, a non-polymer material such as metal or ceramic). In instances where the common material is silicone, the seal ring 202 may be formed of a moulded composite that is a silicone-silicone composite. In instances where the common material is a polymer, the seal ring 202 may be formed of a moulded composite that is a polymer-polymer composite. In instances where the common material is non-polymeric, the seal ring 202 may be formed of a moulded composite that is a ceramic-ceramic composite or a metal-metal composite. In certain instances, the material (e.g., silicone) may be the same, but the composition of the material may be different. For example and as explained in further detail below, the seal ring 202 may be formed of a common material with the first material having a different material (*e*.*g*., stiffness, flexibility) property than the second material.

The seal ring 202, in these instances, may be formed of a composite of two or more portions or two or more sections of the same material type. In addition, the seal ring 202 may be formed of two or more materials that are moulded together. The two or more materials may be formed together using a dual-shot moulding processing. The dual shot moulding process may apply the first material on the second material to form the seal ring 202.

In certain instances, the seal ring 202 may be formed two or more materials that are different materials. For example, the seal ring 202 may be formed of a moulded composite that includes a silicone-polymer composite. In other instances, the seal ring 202 may be formed of a moulded composite that includes a silicone-non-polymeric composite. The non-polymeric material may be a ceramic or metal material. The two or more materials may be formed together using a dual-shot moulding processing. The dual shot moulding process may apply the first material on the second material to form the seal ring 202.

FIG. 2B is a cross-section illustration of the seal ring 202 and core section 204, shown in FIG. 2A, in accordance with various aspects of the present disclosure. As shown, the seal ring 202 includes a substantially smooth outer surface 206. The seal ring 202 being a composite seal ring 202 may facilitate the seal ring 202 having the outer surface 206 without protrusions or other additional structures. The seal ring 202 may be placed within the core section 204 without sticking or without substantial obstructions in arranging the seal ring 202 within the core section 204. In certain instances, the seal ring 202 is moulded to an inner surface 208 of the core section 204. The outer surface 206 of the seal ring 202 may engage the inner surface 208 of the core section 204 to eliminate adulterant, contaminant, or unintended materials (*e*.*g*., epoxy, overmould) from contact with or entering a lumen of a connector bore that the core section 204 forms a part of.

In certain instances, the inner surface 208 of the core section 204 may be substantially uniform. In addition and as shown, the outer surface 206 of the seal ring 202 may be substantially uniform (in addition to being a substantially smooth outer surface 206). The substantially uniform outer surface 206 may be configured to engage the substantially uniform inner surface 208 of the core section 204. In addition, the seal ring 202 may include interior ribs 210, 212 having substantially curved apices. The interior ribs 210 may extend around the interior of the seal ring 202 and be configured to engage a portion of a lead. The seal ring 202 being a moulded composite of two or more materials may facilitate the interior ribs 210, 212 having curved apices as opposed to a more abrupt peak. For example, the seal ring 202 include substantially uniform surfaces 214, 216 on opposing sides of the interior ribs 210, 212. Other prior seal rings may include an abrupt angle change leading to ribs. These prior seal rings may have a greater insertion force for the lead to pass through and engage with the prior seal rings as compared to the seal rings 202 shown and discussed herein. In certain instances, the substantially uniform surfaces 214, 216 may be parallel with the substantially uniform outer surface 206 of the seal ring 202 (which are circumferential about the seal ring 202).

In certain instances, as noted above, the seal ring 202 is moulded to the inner surface 208 of the core section 204. The seal ring 202 may be dual shot moulded into with the inner surface 208 of the core section 204. The first material of the seal ring 202 (silicone) may be moulded to the inner surface 208 of the core section 204 and then the second material of the seal ring 202 may be moulded to the first material. In certain instances, the seal ring 202 being dual shot moulded to the inner surface 208 of the core section 204 may improve the concentricity of the bore alignment, therefore reducing the spread of an insertion force of a lead within the core section 204. In certain instances, the seal ring 202 being moulded to the inner surface 208 of the core section 204 may reduce stack up tolerance to which the seal ring 202 accounts for (in relation to electrical isolation/sealing between one or more seal rings 202 and connector blocks as described in further detail below). Moulding the seal ring 202 to the core section 204 can enable are slight larger inner diameter of the seal ring 202, which reduces insertion force of the lead into the core section 204.

As noted above, the seal ring 202 may be formed of a common material with the first material having a different material (e.g., stiffness, flexibility) property than the second material. In certain instances, the first material may be less flexible than the second material of the seal ring 202. More specifically, the first material may include a high durometer and the second material includes a low durometer. In certain instances, ribs 210, 212 (and the substantially uniform surfaces 214, 216) may be formed of the second material and the outer surface 206 may be formed of the first material. The outer surface 206 being formed of a stiffer material than the ribs 210, 212 (and the substantially uniform surfaces 214, 216) may facilitate maintaining a position of the seal ring 202 within the core section 204. In instances where the seal ring 202 includes two different materials, the outer surface 206 being formed of a stiffer material (e.g., polymer, ceramic, plastic, metal) than the ribs 210, 212 and the substantially uniform surfaces 214, 216 (*e.g*., silicone).

FIG. 3 is a cross-sectional view of another example connector port subassembly medical device, in accordance with various aspects of the present disclosure. The subassembly medical device includes a core subassembly 300, which may be formed from a rigid polymer that is a non-conductive polymer, and a connector bore 302, having a proximal end 304 and a distal end 306, arranged within the core subassembly 300. In addition, one or more connector blocks 308 and one or more seal rings 312 are arranged within the connector bore 302 (which may be formed by core sections as shown in detail in FIG. 2). In certain instances, the connector blocks 308 and/or the seal rings 312 are configured to interference fit within the connector bore 302.

The one or more seal rings 312 and one or more connector blocks 308 may be arranged between the proximal end 304 and distal end 306 of the connector bore 302. In certain instances and as shown, the seal rings 312 include a plurality of seal rings 312a, 312b, 312c and 312d and the one or more connector blocks 308 includes a plurality of connector blocks 308a, 308b, 308c. The number of connector blocks 308a, 308b, 308c and seal rings 312a, 312b, 312c and 312d is dependent on the industry standard desired for the core subassembly 300. As shown, the core subassembly 300 includes three connector blocks 308a, 308b, 308c separated by four seal rings 312a, 312b, 312c and 312d. FIG. 3, for example, shows a connector port subassembly that may be used for the IS-4 and DF-4 standards.

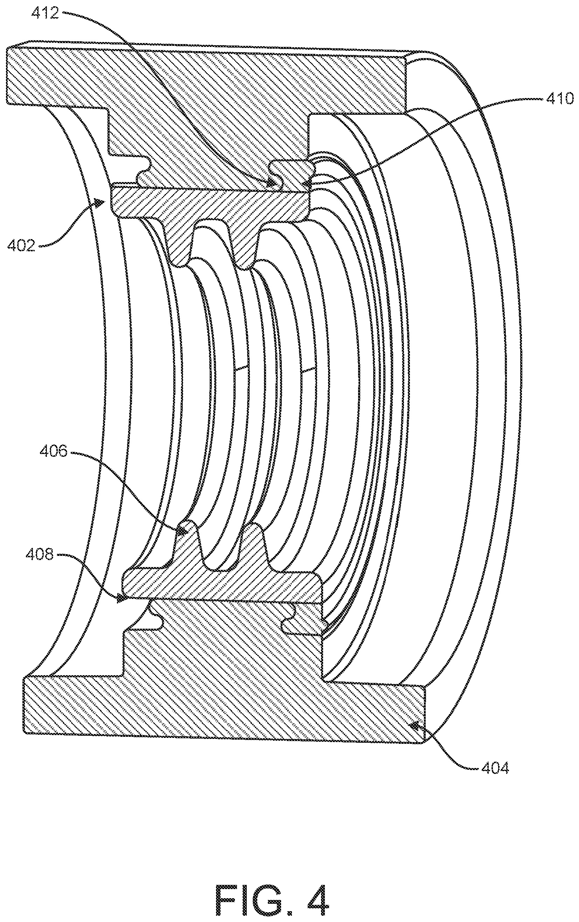

In certain instances, the seal rings 312a, 312b, 312c and 312d include a substantially smooth or uniform outer surface 318. The outer surface 318 of the seal rings 312a, 312b, 312c and 312d engage an inner surface of the connector bore 302 as is described in further detail below with reference to FIG. 4. The core subassembly 300 includes one or more windows 310a, 310b, 310c arranged through the core subassembly 300 to the one or more connector blocks 308. The seal rings 312a, 312b, 312c and 312d may be configured to eliminate adulterant, contaminant, or unintended materials (e.g., epoxy, overmould) from contact with or entering the connector bore 302 (*e*.*g*., lead cavity within the connector blocks 308a, 308b, 308c and seal rings 312a, 312b, 312c and 312d) by way of or through the one or more windows 310a, 310b, 310c.

In certain instances, as noted above, the seal rings 312a, 312b, 312c and 312d are moulded to the inner surface of the connector bore 302. The seal rings 312a, 312b, 312c and 312d may be dual shot moulded into with the inner surface of the connector bore 302. The first material of the seal rings 312a, 312b, 312c and 312d (silicone) may be moulded to the inner surface of the connector bore 302 and then the second material of the seal rings 312a, 312b, 312c and 312d may be moulded to the first material. In certain instances, the seal rings 312a, 312b, 312c and 312d being dual shot moulded to the inner surface of the connector bore 302 may improve the concentricity of the bore alignment, therefore reducing the spread of an insertion force of a lead within the connector bore 302. In certain instances, the seal rings 312a, 312b, 312c and 312d being moulded to the inner surface of the connector bore 302 may reduce stack up tolerance to which the seal rings 312a, 312b, 312c and 312d account for (in relation to electrical isolation/sealing between seal rings 312a, 312b, 312c and 312d and connector blocks 308a, 308b, 308c). Moulding the seal rings 312a, 312b, 312c and 312d to the connector bore 302 can enable are slight larger inner diameter of the seal rings 312a, 312b, 312c and 312d, which reduces insertion force of the lead into the connector bore 302.

In certain instances and as shown in FIG. 1, the header may include multiple connectors for leads. The header may include a first subassembly 300 and a second subassembly 300 (or additional subassemblies 300). In certain instances, forming the header includes overmoulding the first connector port subassembly 300 and the second connector port subassembly 300 to a housing of an implantable medical device, with the first connector port subassembly 300 being arranged above the second connector port subassembly 300 (*e*.*g*., as shown in FIG. 1) or beside the second connector port subassembly 300.

A retaining sleeve 330 may be arranged adjacent a proximal most one of the seal rings 312c, as shown. In other instances, the retaining sleeve 330 may be arranged adjacent a proximal most one of connector blocks 308c. In either instance, the retaining sleeve 330 is arranged proximal to each of the connector blocks 308a, 308b, 308c and seal rings 312a, 312b, 312c. The retaining sleeve 330 may facilitate maintaining a position of the connector blocks 308a, 308b, 308c and seal rings 312a, 312b, 312c within the connector bore 302.

To manufacture the core subassembly 300, the connector blocks 308a, 308b, 308c may be arranged within the connector bore 302 with the seal rings 312a, 312b, 312c being arranged adjacent to and separating the connector blocks 308a, 308b, 308c. The seal rings 312a, 312b, 312c include two or more materials and may be formed from dual shot moulding. The seal rings 312a, 312b, 312c may be placed within the connector bore 302 while reducing adhesion between the connector bore 302 and the outer surface 318 of the seal rings 312a, 312b, 312c. As noted above, the composite seal rings 312a, 312b, 312c may be formed from a dual shot moulding process. In addition, the composite seal rings 312a, 312b, 312c allow for a harder exterior (*e*.*g*., as discussed above with reference to FIG. 2) that allows for stability within the connector bore 302 when assembled into a header.

FIG. 4 is a cross-sectional illustration of an illustration of an example seal ring 402 and core section 404, in accordance with various aspects of the present disclosure. In certain instances, the seal ring 402 includes two or more materials. The seal ring 402 may be formed of a moulded composite of the two or more materials. The two or more materials may include a first material and a second material with the first material and the second material being a common material (*e*.*g*., silicone, a polymer material, a non-polymer material such as metal or ceramic).

In certain instances, the seal ring 402 may be formed two or more materials that are different materials. For example, the seal ring 402 may be formed of a moulded composite that includes a silicone-polymer composite. In other instances, the seal ring 402 may be formed of a moulded composite that includes a silicone-non-polymeric composite. The non-polymeric material may be a ceramic or metal material. The two or more materials may be formed together using a dual-shot moulding processing. The dual shot moulding process may apply the first material on the second material to form the seal ring 402. As discussed in detail above, the seal ring 402 may include ribs 406 and a substantially uniform outer surface 408.

In certain instances, the seal ring 402 includes a first interlock 410. The first interlock 410 may be arranged along a perimeter or end portion of the substantially uniform outer surface 408. The connector bore (by way of the core section 404) may include a second interlock 412. In certain instances, the first interlock 410 and the second interlock 412 are configured to engage to maintain a position of the one or more seal ring 402 within the connector bore. The first interlock 410 and the second interlock 412 may be used in the absence of a bond (*e*.*g*., chemical or adhesive) or in addition to the bond.

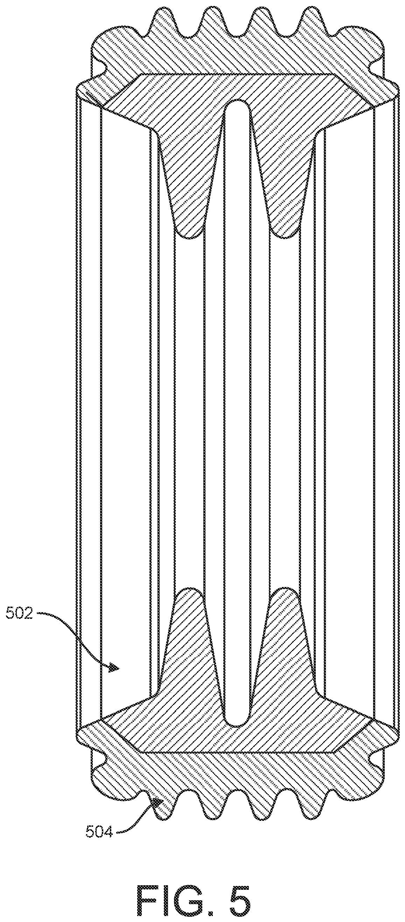

FIG. 5 is a cross-sectional illustration of an illustration of an example seal ring 502, in accordance with various aspects of the present disclosure. As discussed in detail above, the seal ring 502 may be formed of a moulded composite of two or more materials. The two or more materials may include a first material and a second material with the first material and the second material being a common material (*e*.*g*., silicone, a polymer material, a non-polymer material such as metal or ceramic).

In certain instances and as is shown in FIG. 5, the seal ring 502 includes ribs 504 arranged on an outer surface. The ribs 504 may further facilitate eliminating adulterant, contaminant, or unintended materials (*e*.*g*., epoxy, overmould) from contact with or entering a lumen of a connector bore that the seal ring 502 is arranged within. The ribs 504 may be formed of one material and the other portions of the seal ring 502 may be formed of another material. In certain instances, the ribs 504 may be less flexibility or having a greater hardness than other portions of the seal ring 502. In these instances, the seal ring 502 may include a harder exterior (*e.g*., as discussed above with reference to FIG. 2) that allows for stability within the connector bore when assembled into a header.

The present invention is set out in the following claims.

## Claims

1. A connector port subassembly for a medical device, the connector port subassembly comprising:
a connector bore (302, 404) arranged within a core subassembly (300) including a proximal end (304) and a distal end (306);
one or more connector blocks (308a, 308b, 308c) arranged within the connector bore (302); and
one or more seal rings (312a, 312b, 312c, 312d, 402, 502) moulded to an interior surface of the connector bore (302, 404) and arranged adjacent to the one or more connector blocks (308a, 308b, 308c) , wherein the one or more seal rings (312a, 312b, 312c, 312d, 402, 502) comprise a dual-shot moulded composite comprising a first material and a second material,
and wherein
the first material of each of the one or more seal rings (312a, 312b, 312c, 312d) is moulded to the interior surface of the connector bore (302, 404), and the second material of each of the one or more seal rings (312a, 312b, 312c and 312d) is moulded to the first material.

2. The connector port subassembly of claim 1, wherein the first material and the second material comprise a common material.

3. The connector port subassembly of claim 2, wherein the common material is silicone and the moulded composite comprises a silicone-silicone composite.

4. The connector port subassembly of claim 1, wherein the first material and the second material comprise different materials.

5. The connector port subassembly of claim 4, wherein the first material is silicone and the second material is a polymer and the moulded composite comprises a silicone- polymer composite.

6. The connector port subassembly of claim 4, wherein the first material is silicone and the second material is a non-polymeric material and the moulded composite comprises a silicone-non-polymeric composite.

7. The connector port subassembly of any one of claims 1-6, wherein the one or more seal rings include a substantially smooth outer surface.

8. The connector port subassembly of any one of claims 1-7, wherein the one or more seal rings (402) include interior ribs (406) having substantially curved apices.

9. The connector port subassembly of any one of claims 1-8, wherein the one or more seal rings (402) include a first interlock (410) and the connector bore (404) includes a second interlock (412), and the first interlock (410) and the second interlock (412) are configured to engage to maintain a position of the one or more seal (402) rings within the connector bore (404).

10. A method of manufacturing one or more connector port subassemblies , the method comprising:
arranging one or more connector blocks (308a, 308b, 308c) within a connector bore (302, 404), the connector bore (302, 404) being arranged within a core subassembly(300) and having a proximal end (304) and a distal end (306); and
moulding one or more seal rings (312a, 312b, 312c, 312d, 402, 502) comprising a first material and a second material to an interior surface of the connector bore (302, 404) adjacent to the one or more connector blocks (308a, 308b, 308c),
wherein moulding one or more seal rings (312a, 312b, 312c, 312d, 402, 502) comprises moulding the first material to the interior surface of the connector bore (302, 404), and moulding the second material to the first material in a dual-shot moulding process.

11. The method of claim 10, wherein the first material and the second material comprise a common material.

12. The method of claim 10, wherein the first material and the second material comprise different materials.

13. The method of any one of claims 10-12, wherein the one or more seal rings (312a, 312b, 312c, 312d, 402, 502) include a substantially smooth outer surface, interior ribs (406) having substantially curved apices, and substantially uniform surfaces on opposing sides of the interior ribs (406).

## Patentansprüche

1. Steckeranschluss-Teilanordnung für eine medizinische Vorrichtung, wobei die Steckeranschluss-Teilanordnung aufweist:
eine in einer Kern-Teilanordnung (300) angeordnete Steckerbohrung (302, 404) mit einem proximalen Ende (304) und einem distalen Ende (306);
einen oder mehrere Steckerblöcke (308a, 308b, 308c), die in der Steckerbohrung (302) angeordnet sind; und
einen oder mehrere Dichtungsringe (312a, 312b, 312c, 312d, 402, 502), die an eine Innenfläche der Steckerbohrung (302, 404) geformt sind und angrenzend an den einen oder die mehreren Steckerblöcke (308a, 308b, 308c) angeordnet sind, wobei der eine oder die mehreren Dichtungsringe (312a, 312b, 312c, 312d, 402, 502) einen doppelschuss-geformten Verbundwerkstoff aufweisen, der ein erstes Material und ein zweites Material aufweist, und wobei das erste Material jedes des einen oder der mehreren Dichtungsringe (312a, 312b, 312c, 312d) an die Innenfläche der Steckerbohrung (302, 404) geformt ist, und das zweite Material jedes des einen oder der mehreren Dichtungsringe (312a, 312b, 312c, 312d) an das erste Material geformt ist.

2. Steckeranschluss-Teilanordnung nach Anspruch 1, wobei das erste Material und das zweite Material ein gemeinsames Material aufweisen.

3. Steckeranschluss-Teilanordnung nach Anspruch 2, wobei das gemeinsame Material Silikon ist und der geformte Verbundwerkstoff einen Silikon-Silikon-Verbundwerkstoff aufweist.

4. Steckeranschluss-Teilanordnung nach Anspruch 1, wobei das erste Material und das zweite Material unterschiedliche Materialien aufweisen.

5. Steckeranschluss-Teilanordnung nach Anspruch 4, wobei das erste Material Silikon ist und das zweite Material ein Polymer ist, und der geformte Verbundwerkstoff einen SilikonPolymer-Verbundwerkstoff aufweist.

6. Steckeranschluss-Teilanordnung nach Anspruch 4, wobei das erste Material Silikon ist und das zweite Material ein nicht-polymeres Material ist, und der geformte Verbundwerkstoff einen Silikon-Nichtpolymer-Verbundwerkstoff aufweist.

7. Steckeranschluss-Teilanordnung nach einem der Ansprüche 1 bis 6, wobei der eine oder die mehreren Dichtungsringe eine im Wesentlichen ebene Außenfläche aufweisen.

8. Steckeranschluss-Teilanordnung nach einem der Ansprüche 1 bis 7, wobei der eine oder die mehreren Dichtungsringe (402) Innenrippen (406) aufweisen, die im Wesentlichen bogenförmige Scheitel haben.

9. Steckeranschluss-Teilanordnung nach einem der Ansprüche 1 bis 8, wobei der eine oder die mehreren Dichtungsringe (402) eine erste Eingriffseinrichtung (410) aufweisen und die Steckerbohrung (404) eine zweite Eingriffseinrichtung (412) aufweist, und die erste Eingriffseinrichtung (410) und die zweite Eingriffseinrichtung (412) konfiguriert sind, ineinander zu greifen, um den einen oder die mehreren Dichtungsringe (402) in der Steckerbohrung (404) in Position zu halten.

10. Verfahren zur Herstellung einer oder mehrerer Steckeranschluss-Teilanordnungen, wobei das Verfahren aufweist:
Anordnen eines oder mehrerer Steckerblöcke (308a, 308b, 308c) in einer Steckerbohrung (302, 404), wobei die Steckerbohrung (302, 404) in einer Kern-Teilanordnung (300) angeordnet ist und ein proximales Ende (304) und ein distales Ende (306) hat; und
Formen eines oder mehrerer Dichtungsringe (312a, 312b, 312c, 312d, 402, 502), die ein erstes Material und ein zweites Material aufweisen, an eine Innenfläche der Steckerbohrung (302, 404) angrenzend an den einen oder die mehreren Steckerblöcke (308a, 308b, 308c),
wobei Formen eines oder mehrerer Dichtungsringe (312a, 312b, 312c, 312d, 402, 502) aufweist: Formen des ersten Materials an die Innenfläche der Steckerbohrung (302, 404) und Formen des zweiten Materials an das erste Material in einem Doppelschuss-Formprozess.

11. Verfahren nach Anspruch 10, wobei das erste Material und das zweite Material ein gemeinsames Material aufweisen.

12. Verfahren nach Anspruch 10, wobei das erste Material und das zweite Material unterschiedliche Materialien aufweisen.

13. Verfahren nach einem de Ansprüche 10 bis 12, wobei der eine oder die mehreren Dichtungsringe (312a, 312b, 312c, 312d, 402, 502) eine im Wesentlichen ebene Außenfläche, Innenrippen (406) mit im Wesentlichen bogenförmigen Scheiteln und im Wesentlichen gleichförmige Oberflächen an entgegengesetzten Seiten der Innenrippen (406) aufweisen.

## Revendications

1. Sous-assemblage de ports de connecteur pour un dispositif médical, le sous-assemblage de ports de connecteur (300) comprenant :
un alésage de connecteur (302, 404) disposé à l'intérieur d'un sous-assemblage central (300) incluant une extrémité proximale (304) et une extrémité distale (306) ;
un ou plusieurs blocs de connecteur (308a, 308b, 308c) disposés à l'intérieur de l'alésage de connecteur (302) ; et
une ou plusieurs bagues d'étanchéité (312a, 312b, 312c, 312d, 402, 502) moulées contre une surface intérieure de l'alésage de connecteur (302, 404) et disposées adjacentes aux un ou plusieurs blocs de connecteur (308a, 308b, 308c), lesquelles une ou plusieurs bagues d'étanchéité (312a, 312b, 312c, 312d, 402, 502) comprennent un composite moulé par injection double comprenant un premier matériau et un deuxième matériau,
et dans lequel le premier matériau de chacune des une ou plusieurs bagues d'étanchéité (312a, 312b, 312c, 312d) est moulé contre la surface intérieure de l'alésage de connecteur (302, 404), et le deuxième matériau de chacune des une ou plusieurs bagues d'étanchéité (312a, 312b, 312c et 312d) est moulé contre le premier matériau.

2. Sous-assemblage de ports de connecteur selon la revendication 1, dans lequel le premier matériau et le deuxième matériau comprennent un matériau commun.

3. Sous-assemblage de ports de connecteur selon la revendication 2, dans lequel le matériau commun est la silicone et le composite moulé comprend un composite de silicone-silicone.

4. Sous-assemblage de ports de connecteur selon la revendication 1, dans lequel le premier matériau et le deuxième matériau comprennent des matériaux différents.

5. Sous-assemblage de ports de connecteur selon la revendication 4, dans lequel le premier matériau est la silicone et le deuxième matériau est un polymère et le composite moulé comprend un composite de silicone-polymère.

6. Sous-assemblage de ports de connecteur selon la revendication 4, dans lequel le premier matériau est la silicone et le deuxième matériau est un matériau non polymère et le composite moulé comprend un composite de silicone-non polymère.

7. Sous-assemblage de ports de connecteur selon l'une quelconque des revendications 1 à 6, dans lequel la ou les bagues d'étanchéité comprennent une surface extérieure sensiblement lisse.

8. Sous-assemblage de ports de connecteur selon l'une quelconque des revendications 1 à 7, dans lequel la ou les bagues d'étanchéité (402) comprennent des nervures intérieures (406) ayant des apex sensiblement courbés.

9. Sous-assemblage de ports de connecteur selon l'une quelconque des revendications 1 à 8, dans lequel la ou les bagues d'étanchéité (402) comprennent un premier verrouillage (410) et l'alésage de connecteur (404) comprend un deuxième verrouillage (412), et le premier verrouillage (410) et le deuxième verrouillage (412) sont configurés pour s'engager de façon à maintenir la position de la ou des bagues d'étanchéité (402) à l'intérieur de l'alésage de connecteur (404).

10. Procédé de fabrication d'un ou plusieurs sous-assemblages de ports de connecteur, le procédé comprenant :
la disposition d'un ou plusieurs blocs de connecteur (308a, 308b, 308c) à l'intérieur d'un alésage de connecteur (302, 404), l'alésage de connecteur (302, 404) étant disposé à l'intérieur d'un sous-assemblage central (300) et ayant une extrémité proximale (304) et une extrémité distale (306) ; et
le moulage d'une ou plusieurs bagues d'étanchéité (312a, 312b, 312c, 312d, 402, 502) comprenant un premier matériau et un deuxième matériau contre une surface intérieure de l'alésage de connecteur (302, 404), adjacentes aux un ou plusieurs blocs de connecteur (308a, 308b, 308c),
dans lequel le moulage d'une ou plusieurs bagues d'étanchéité (312a, 312b, 312c, 312d, 402, 502) comprend le moulage du premier matériau contre la surface intérieure de l'alésage de connecteur (302, 404), et le moulage du deuxième matériau contre le premier matériau selon un procédé de moulage par injection double.

11. Procédé selon la revendication 10, dans lequel le premier matériau et le deuxième matériau comprennent un matériau commun.

12. Procédé selon la revendication 10, dans lequel le premier matériau et le deuxième matériau comprennent des matériaux différents.

13. Procédé selon l'une quelconque des revendications 10 à 12, dans lequel la ou les bagues d'étanchéité (312a, 312b, 312c, 312d, 402, 502) comprennent une surface extérieure sensiblement lisse, des nervures intérieures (406) ayant des apex sensiblement courbes, et des surfaces sensiblement uniformes sur des côtés opposés des nervures intérieures (406).
